# EUROPEAN PATENT SPECIFICATION

(11) **EP 2 694 942 B1**
(45) Date of publication and mention of the grant of the patent: **19.07.2017**
(21) Application number: 12720264.6
(22) Date of filing: 10.04.2012
(51) Int. Cl.: G01N 1/30, C12Q 1/24, G01N 35/00

(54) **Method for preparing cytological specimens for analyses**
Verfahren zur Vorbereitung von zytologischen proben für Analysen
Procédé pour préparer des échantillons cytologiques pour des analyses

(30) Priority: 06.04.2011 GB 201105789
(43) Date of publication of application: 12.02.2014
(73) Proprietor: Aremu, Peters, Orpington, Kent BR6 9XL (GB)
(72) Inventor: Aremu, Peters, Orpington, Kent BR6 9XL (GB)
(74) Representative: Games, Robert Harland
(86) International application number: PCT/GB2012/050785
(87) International publication number: WO 2012/137019

(56) References cited:
- EP-A1- 1 422 509
- EP-A2- 1 701 148
- GB-A- 2 455 204
- US-A- 6 076 583
- US-A1- 2005 070 020

## Description

The present invention relates to a method for preparing pre-analysis cytological specimens.

Cytology is the study and analysis of the structure, formation, and function of cells. Healthy cells often display certain physical and physiological features that are characteristic of its specific cell type and which differentiate them from other cell types. These can be, for example, the shape and size of the cell, or its interaction with cells of the same type and of different types. By carrying out a visual examination of a specimen of a patient's cells, usually under a microscope, medical or health professionals are able to make diagnoses and evaluations of the patient's condition, based on any abnormalities observed. This is commonly known as diagnostic cytology.

Cytological samples can be obtained in a variety of techniques. A sample can be carried out by scraping or swabbing an area, such as the interior of a mouth. Fine needle aspiration can also be carried out by using a needle to aspirate material from palpable swelling of pathological suspicion and/or less accessible areas such as the lungs, liver, or bones.

Gynaecological samples, such as gynaecological cells obtained during a cervical smear test, are one of the most common types of cell studied in diagnostic cytology. Traditionally, a sample obtained from the cervix is smeared directly onto a microscope slide and then fixed with an alcohol based fixative before being stained with Papanicolaou stain. This is commonly known as a 'Pap smear', or a 'Pap test'. The conventional screening method can sometimes produce inadequate smears, where an insufficient quantity of cells are transferred to the slide, and excessive blood and mucus on the slide makes diagnosis difficult.

In recent years, a different technique has been used increasingly, in which a sample of cells to be studied is placed into a vial containing liquid preservative. Once in the laboratory, the sample is agitated to achieve an even distribution of cells. The sample is then collected from the vial, any exudates, blood, or mucus removed by centrifugation or blended in a high-speed spin, and the cells spread out on a slide. The smeared cells are then fixed and stained ready for examination.

Non-gynaecological samples, such as urine, pleural fluid and ascetic fluid can also be Pap stained for microscopic examination. Other staining techniques are also used for non-gynaecological samples, such as the May-Grunwald staining technique, Periodic acid-Schiff staining, and Gram staining. The May-Grunwald technique, which is a peer staining technique to Pap testing, usually requires the specimens to be air dried and not fixed by an alcohol based fixative. The current practice of collecting a sample for such staining techniques is to place the sample in a blank container with no fixative or preservative. Without the May-Grunwald staining technique, non-gynaecological cell investigations are at risk of misinterpretation or less confident diagnoses.

Equipment is currently available that automates the staining of gynaecological samples. However, there is currently no equipment available that fully automates the processing of staining techniques other than Pap staining. Non-Pap staining techniques are conventionally carried out at least partially manually. However, the success of manual intervention depends on the experience level of the individual user. Inexperience can lead to inadequately prepared specimens being smeared and stained for analysis.

Furthermore, currently, for a sample that requires more than one type of staining technique, each staining technique needs to be processed separately. Sometimes this means the sample may need to be prepared by different machines or prepared manually making processing more tedious and inconsistent. This means that more than one sample may need to be collected in order to provide a sufficient quantity for processing using the different techniques and/or for sending to the different laboratories. This can increase the cost and opportunities for errors, not to mention the stress and discomfort a patient may experience with the collection of repeated samples if such preparations turn out to be inadequate.

Although there are automated processors available for preparing Pap stains, if the sample also requires other staining techniques, the non Pap stains will still need to be processed at least partially manually. Should the medical professional wish to examine all of the slides at the same time in order to gather a comprehensive overview of the health of the patient, he or she needs to wait until all the slides have been processed. Consequently, this means that automating the processing of Pap staining may not actually produce any time efficiencies.

According to the invention, there is provided a method of preparing cytological specimens for analysis, which comprises the steps of: providing cytological material in a specimen container, adding anti-coagulant preservative to the specimen container, and receiving the cytological material and anti-coagulant in an automated apparatus for pre-analysis preparation. The anti-coagulant preservative is selected to inhibit cellular activity and maintain cellular morphology without killing cells. The method of the invention is defined in claim 1. The benefit of this method is that the cytological cells are preserved in an active state, with little or no degeneration prior to staining. Use of an anti-coagulant preservative in the preparation of cytological specimens leads to minimal damage or distortion to the structure, and architectural and/or morphological appearances of the cytological cells, as tends to occur using an alcohol based preservative. Cells subjected to an alcohol based preservative become fixed and effectively die, making the cells difficult to manipulate for successful performance in a wide range of diagnostic techniques.

Cells harvested from the body and preserved using the anti-coagulant and processed in this manner perform better in any subsequent treatment they are put through. Histochemical tests, PAS, GMS, ZN and Perl's Prussian Blue and ancillary tests such as HPV testing, Immunocytochemical tests, flow-cytometry and the like can be performed on harvested cells with an excellent staining outcome.

Preferable and/or optional features of the invention are set forth in dependent claims 2 to 15. The present invention relates to a method of preserving cellular material comprising the steps of a) placing the cellular material in a container, b) adding to the container a solution comprising 50ml heparin sodium, 50ml saline, 5g chloral hydrate and 0.1g citric acid, or any other quantities substantially in those proportions, and c) freezing the cellular material suspended in the solution.

Traditionally, FNA samples are taken with a conventional syringe and it is essential to expel any excess fluid taken quickly to avoid clotting of the sample. Clots frequently occur within a short period of time, and the smaller the sample, the higher the risk of clotting. Sometimes, the aspirated samples clot immediately and get stuck in the barrel, rendering the sample useless.

The specially adapted syringe is internally coated with heparin, which would prevent clotting in any form. No matter how small the sample, cells in the syringe can be still be successfully harvested and as much as possible be presented in their morphological state.

In use, cytological material is provided in a specimen container at point of patient contact. Anti-coagulant is added to the cytological material. The anti-coagulant acts as a preservative to the cytological material whilst the sample is being transported to an apparatus for pre-cytodiagnosis preparation. Anti-coagulants which may be used include: 1) heparin, which is used to preserve urine, peritoneal ascetic fluid and ovarian fluid, 2) EDTA, which is used to preserve mucous and fibrinoid samples e.g. sputum, bronchial larvage, washings, and any other sample that has a heavy texture or viscosity, and 3) a combination of heparin sodium, chloral hydrate and citric acid. Heparin sodium choral hydrate is a cytoplasmic preservative and citric acid is a nuclear sharpener. It is found that a mixture comprising 50ml heparin sodium, 50ml saline, 5g choral hydrate and 0.1g citric acid forms a preservative suitable for preserving cells in urine and serous fluids whilst preventing clotting of the extracellular medium surrounding the cells. EDTA prevents coagulation as well as degrading fibrin and mucous without destroying the cells of interest.

The specimen container may be pre-charged with the anti-coagulant or the anti-coagulant may be added to the container after the cytological material is inserted therein.

The specimen container is then tagged using identification means so that the identity of the specimen container is unique and traceable. The identification means typically labels the specimen container with a barcode. The identification means assists with the identification and traceability of a sample from the initial specimen container to the final specimen slide ready for cytodiagnosis.

The medical professional optionally categorizes the cytological material according to the viscosity and/or level of blood staining, depending on the nature of the sample. This information relating to the sample is entered into a log, for example, on the personal computer.

Where the quantity of cells collected is greater than the quantity needed to produce the required smears, the excess cells may be stored for future use.

Excess cells may be stored in 1% formal saline, comprising 0.9g sodium chloride, 99ml distilled water and 1ml concentrated formaldehyde. A small amount of this solution is dispensed on top of the deposit to form a suspension, and the suspension may be kept for at least two years in a plastic container.

Alternatively, excess cells may be freeze-dried in citreous heparin, comprising 50ml heparin sodium, 50ml saline, 5g chloral hydrate and 0.1g citric acid. After being mixed with citreous heparin, the deposit is frozen, and is preserved for around one year.

## Claims

1. A method of preparing cytological specimens for analysis, which comprises the steps of:
a. Providing cytological material in a specimen container,
b. Adding anti-coagulant preservative to the specimen container, where the anti-coagulant preservative comprises at least in part of heparin sodium chloral hydrate, and at least in part of citric acid,
c. Receiving the cytological material and anti-coagulant in an automated apparatus for pre-analysis preparation,
the anti-coagulant preservative being selected to inhibit cellular activity and maintain cellular morphology without killing cells.

2. A method as claimed in claim 1, in which the anti-coagulant preservative comprises substantially 50ml heparin sodium, 50ml saline, 5g chloral hydrate, and 0.1g citric acid or any other quantities substantially in that proportion.

3. A method as claimed in claim 1 or 2, in which the anti-coagulant is added to the specimen container before the cytological material is provided therein.

4. A method as claimed in any one of claims 1 to 3, further comprising the step of transferring the cytological material and anti-coagulant from the specimen container to a preparation receptacle, the receptacle having a fluid inlet for receiving diluents and a fluid outlet for draining supernatant, where each receptacle has a flexible second said receptacle detachably attached at one end of the first said receptacle.

5. A method as claimed in claim 4, further comprising the step of purifying the cytological material in a purification procedure to remove unwanted non-epithelial materials.

6. A method as claimed in claim 5, in which said purification procedure includes detecting the quantity of mucous, red blood cells, neutrophils and epithelial cells in the cytological material.

7. A method as claimed in claim 6, in which the purification procedure includes adding one or more of the following agents to the first receptacle in response to the quantity of mucous, red blood cells, neutrophils and epithelial cells detected: a mucous withdrawing agent, a red blood cell removing agent, and a neutrophils removing agent.

8. A method as claimed in claim 7, in which the purification procedure includes monitoring the quantity of mucous, red blood cells and neutrophils in the first receptacle after the one or more agents have been added and determining the point at which the mucous, red blood cells and neutrophils become absent.

9. A method as claimed in claim 8, in which the purification procedure includes detecting the quantity of epithelial material remaining in the first receptacle and/or establishing whether a minimum quantity of epithelial material is present.

10. A method as claimed in any one of claims 5 to 9, in which the purification procedure includes adding diluents to the first receptacle through the fluid inlet for reducing the viscosity of fluids therewithin.

11. A method as claimed in any one of claims 5 to 10, in which the purification procedure includes inserting the first and second receptacles into a carousel and rotating the carousel such that fluids within the first receptacle undergo centrifugation.

12. A method as claimed in claim 11, in which the purification procedure includes draining supernatant from the first receptacle through the fluid outlet to leave epithelial cells in the first receptacle, and transferring residual epithelial cells from the first receptacle to the second receptacle through a filter.

13. A method as claimed in claim 12, further comprising the step in a smearing unit of smearing a portion of the epithelial cells from the second receptacle onto a specimen slide, where the horizontal extent of said smeared epithelial cells is adjusted depending on the cellularity of the sample and the thickness of said smeared epithelial cells is controlled to less than approximately 5 micrometres.

14. A method as claimed in claim 13, further comprising the step of fixing said smeared deposit on the slide using a chemical fixing agent and/or using an air-dryer for drying the epithelial cells on the specimen slide.

15. A method as claimed in claim 14, further comprising the step of staining the fixed epithelial cells using a Papanicolaou staining technique or a non-Papanicolaou staining technique, where the non-Papanicolaou staining technique is any one of the following: May Grunwald Giemsa (MGG), Periodic Acid Schiffs (PAL), Peris' Prussian Blue (Perl's), Gomoris Methanamine Silver (GMS), Groscott's methods for Silver, Alcian Blue, Ziehl-Neelson (ZN for Acid Fast), Oil Red O.

## Patentansprüche

1. Methode zur Vorbereitung zytologischer Proben für die Analyse, die die folgenden Schritte umfasst:
a. Bereitstellung des zytologischen Materials in einem Probenbehälter,
b. Hinzufügen von gerinnungshemmendem Konservierungsmittel zum Probenbehälter, wobei das gerinnungshemmende Konservierungsmittel zumindest teilweise Heparin-Natrium-Chloralhydrat und zumindest teilweise Zitronensäure umfasst,
c. Aufnahme des zytologischen Materials und des Gerinnungshemmers in einer automatisierten Vorrichtung für die Vorbereitung vor der Analyse,
wobei das gerinnungshemmende Konservierungsmittel ausgewählt wird, um die Zellaktivität zu hemmen und die zelluläre Morphologie ohne Tötung von Zellen zu erhalten.

2. Methode nach Anspruch 1, bei der das gerinnungshemmende Konservierungsmittel wesentlich 50 ml Heparin-Natrium, 50 ml Kochsalzlösung, 5 g Chloralhydrat und 0,1 g Zitronensäure oder jede andere Menge wesentlich in diesem Anteil umfasst.

3. Methode nach Anspruch 1 oder 2, bei der der Gerinnungshemmer zum Probenbehälter hinzugefügt wird, bevor das zytologische Material darin bereitgestellt wird.

4. Methode nach einem der Ansprüche 1 bis 3, die weiter den Schritt der Übertragung des zytologischen Materials und des Gerinnungshemmers aus dem Probenbehälter zu einem Vorbereitungsgefäß umfasst, wobei das Gefäß über einen Flüssigkeitseinlass für die Aufnahme von Verdünnungsmitteln und einen Flüssigkeitsauslass für das Ablassen von Überstand verfügt, wobei jedes Gefäß über ein flexibles zweites genanntes Gefäß verfügt, das am Ende des ersten genannten Gefäßes abnehmbar befestigt ist.

5. Methode nach Anspruch 4, die weiter den Schritt der Reinigung des zytologischen Materials in einem Reinigungsverfahren zur Entfernung unerwünschter nicht-epithelialer Materialien umfasst.

6. Methode nach Anspruch 5, bei der das genannte Reinigungsverfahren die Erkennung der Menge an Schleim, roten Blutkörperchen, Neutrophilen und Epithelzellen im zytologischen Material beinhaltet.

7. Methode nach Anspruch 6, bei der das Reinigungsverfahren das Hinzufügen eines oder mehrerer der folgenden Agenzien zum ersten Gefäß in Reaktion auf die Menge an erkanntem Schleim, erkannten roten Blutkörperchen, Neutrophilen und Epithelzellen beinhaltet: Ein schleimentziehendes Agens, ein rote Blutkörperchen entfernendes Agens und ein Neutrophile entfernendes Agens.

8. Methode nach Anspruch 7, bei der das Reinigungsverfahren die Überwachung der Menge an Schleim, roten Blutkörperchen und Neutrophilen im ersten Gefäß, nachdem ein Agens oder mehrere Agenzien hinzugefügt wurde(n), und die Bestimmung des Punkts, an dem der Schleim, die roten Blutkörperchen und Neutrophilen abwesend werden, beinhaltet.

9. Methode nach Anspruch 8, bei der das Reinigungsverfahren die Erkennung der Menge des im ersten Gefäß verbleibenden epithelialen Materials und/oder die Feststellung, ob eine Mindestmenge an epithelialem Material vorhanden ist, beinhaltet.

10. Methode nach einem der Ansprüche 5 bis 9, bei der das Reinigungsverfahren zur Reduzierung der Viskosität von Flüssigkeiten darin das Hinzufügen von Verdünnungsmitteln zum ersten Gefäß durch den Flüssigkeitseinlass beinhaltet.

11. Methode nach einem der Ansprüche 5 bis 10, bei dem das Reinigungsverfahren das Einfügen des ersten und zweiten Gefäßes in ein Karussell und das Drehen des Karussells, sodass Flüssigkeiten im ersten Gefäß einer Zentrifugation unterzogen werden, beinhaltet.

12. Methode nach Anspruch 11, bei dem das Reinigungsverfahren das Ablassen des Überstands aus dem ersten Gefäß durch den Flüssigkeitsauslass, um die Epithelzellen im ersten Behälter zu belassen, und die Übertragung der verbleibenden Epithelzellen aus dem ersten Gefäß in das zweite Gefäß durch einen Filter beinhaltet.

13. Methode nach Anspruch 12, die weiter in einer Ausstricheinheit den Schritt des Ausstreichens eines Teils der Epithelzellen aus dem zweiten Gefäß auf einen Objektträger umfasst, wobei die horizontale Ausdehnung der genannten ausgestrichenen Epithelzellen in Abhängigkeit von der Zellularität der Probe und der Dicke der genannten ausgestrichenen Epithelzellen auf weniger als etwa 5 Mikrometer angepasst wird.

14. Methode nach Anspruch 13, die weiter den Schritt der Fixierung der ausgestrichenen Ablagerung auf dem Objektträger mit einem chemischen Fixiermittel und/oder mit einem Lufttrockner zum Trocknen der epithelialen Zellen auf dem Objektträger umfasst.

15. Methode nach Anspruch 14, die weiter den Schritt der Färbung der fixierten Epithelzellen mit einer Papanicolaou-Färbetechnik oder einer Nicht-Papanicolaou-Färbetechnik umfasst, wobei die Papanicolaou-Färbetechnik eines des Folgenden ist: May-Grünwald-Giemsa (MGG), Perjodsäure-Schiff (PAS), Preußischblau nach Perl (nach Perl), Gomori-Methenaminsilber (GMS), Groscott-Methoden für Silber, Alcian-Blau, Ziehl-Neelson (ZN für säurefest), Oil Red O.

## Revendications

1. Procédé destiné à la préparation d'échantillons cytologiques à des fins d'analyse, qui comprend les étapes consistant à :
a. placer le matériel cytologique dans un récipient à échantillons,
b. ajouter un agent de conservation anticoagulant dans le récipient à échantillons, dans lequel l'agent de conservation anticoagulant comprend au moins en partie de l'hydrate de chloral, de l'héparine sodique et au moins en partie de l'acide citrique,
c. recevoir le matériel cytologique et l'anticoagulant dans un dispositif automatisé pour une préparation de pré-analyse,
l'agent de conservation anticoagulant étant choisi pour inhiber l'activité cellulaire et maintenir la morphologie cellulaire sans tuer de cellules.

2. Procédé selon la revendication 1, dans laquelle l'agent de conservation anticoagulant comprend sensiblement 50 ml d'héparine sodique, 50 ml de saline, 5 g d'hydrate de chloral, et 0,1 g d'acide citrique ou toute autre quantité sensiblement dans ces proportions.

3. Procédé selon l'une des revendications 1 ou 2, dans lequel l'anticoagulant est ajouté au récipient à échantillons avant introduction du matériel cytologique.

4. Procédé selon l'une quelconque des revendications de 1 à 3, comprenant en outre l'étape consistant à transférer le matériel cytologique et l'anticoagulant du récipient à échantillon au dispositif de réception pour préparation, le dispositif de réception ayant une admission de fluide destinée à recevoir des diluants et une sortie de fluide destinée à drainer le surnageant, chaque dispositif de réception ayant un deuxième dit dispositif de réception flexible fixé de manière amovible à une extrémité du premier dispositif de réception.

5. Procédé selon la revendication 4, comprenant en outre une étape consistant à purifier le matériel cytologique au cours d'une procédure de purification permettant d'éliminer des matières épithéliales indésirables.

6. Procédé selon la revendication 5, dans lequel ladite procédure de purification inclut la détection de la quantité de cellules muqueuses, de globules rouges, de neutrophiles et de cellules épithéliales dans le matériel cytologique.

7. Procédé selon la revendication 6, dans lequel la procédure de purification inclut l'ajout d'un ou de plusieurs des agents suivants dans le premier dispositif de réception en réponse à la quantité de cellules muqueuses, de globules rouges, de neutrophiles et de cellules épithéliales détectée : un agent de retrait des cellules muqueuses, un agent d'élimination des globules rouges et un agent d'élimination des neutrophiles.

8. Procédé selon la revendication 7, dans lequel la procédure de purification inclut le contrôle de la quantité de cellules muqueuses, de globules rouges et de neutrophiles dans le dispositif de réception après l'ajout d'un ou de plusieurs agents et la détermination du point auquel les cellules muqueuses, les globules rouges et les neutrophiles disparaissent.

9. Procédé selon la revendication 8, dans lequel la procédure de purification inclut la détection de la quantité de matériel épithélial restante dans le premier dispositif de réception et / ou la définition de la présence ou non d'une quantité minimale de matériel épithélial.

10. Procédé selon l'une quelconque des revendications 5 à 9, dans lequel la procédure de purification inclut l'ajout de diluants au premier dispositif de réception à travers l'admission de fluide destinée à réduire la viscosité des fluides en son sein.

11. Procédé selon l'une quelconque des revendications 5 à 10, dans lequel la procédure de purification inclut l'insertion des premier et deuxième dispositifs de réception dans un carrousel et la rotation du carrousel de sorte à soumettre les fluides dans le premier dispositif de réception à une centrifugation.

12. Procédé selon la revendication 11, dans lequel la procédure de purification inclut le drainage du surnageant du premier dispositif de réception à travers la sortie de fluide pour laisser les cellules épithéliales dans le premier récipient, et le transfert des cellules épithéliales résiduelles du premier dispositif de réception au second dispositif de réception à travers un filtre.

13. Procédé selon la revendication 12, comprenant en outre l'étape consistant, au sein d'une unité destinée à réaliser des frottis, à préparer un frottis d'une partie des cellules épithéliales provenant du deuxième réceptacle sur une lamelle d'échantillons, l'étendue horizontale desdites cellules épithéliales préparées en frottis est ajustée en fonction de la cellularité de l'échantillon et l'épaisseur desdites cellules épithéliales à moins de 5 micromètres environ étant contrôlée.

14. Procédé selon la revendication 13, comprenant en outre l'étape consistant à fixer ledit dépôt préparé en frottis sur la lamelle à l'aide d'un agent fixateur chimique et / ou à l'aide d'un dessiccateur destiné à sécher les cellules épithéliales sur la lame d'échantillons.

15. Procédé selon la revendication 14, comprenant en outre l'étape consistant à colorer les cellules épithéliales fixées à l'aide d'une coloration de Papanicolaou ou une technique de coloration autre que celle de Papanicolaou, l'autre technique de coloration autre que celle de Papanicolaou étant l'une quelconque des suivantes : May-Grünwald Giemsa (MGG), acide périodique de Schiff (PAS), bleu de Prusse de Perl, méthénamine d'argent de Gomori (GMS), méthénamine d'argent de Grocott, bleu alcian, coloration de Ziehl-Neelsen (ZN pour l'acide rapide), coloration à l'huile rouge O.
